# FASCICULE DE BREVET EUROPEEN

(11) **EP 1 926 458 B1**
(45) Date de publication et mention de la délivrance du brevet: **02.02.2011**
(21) Numéro de dépôt: 06808194.2
(22) Date de dépôt: 22.09.2006
(51) Int. Cl.: A61F 13/00, A61F 13/02

(54) **DISPOSITIF, BANDE ET VETEMENT DE TRAITEMENT D'AFFECTIONS TISSULAIRES CUTANEES ET SOUS-CUTANEES ET DE REPARATION SPORTIVE, ET LEUR PROCEDE DE FABRICATION**
VORRICHTUNG, GURT UND KLEIDUNGSSTÜCK ZUR BEHANDLUNG VON HAUT- UND UNTERHAUTGEWEBE-ERKRANKUNGEN UND ZUR REPARATUR VON SPORTVERLETZUNGEN, UND HERSTELLUNGSVERFAHREN DAFÜR
DEVICE, STRAP AND GARMENT FOR TREATING SKIN AND SUBCUTANEOUS TISSUE DISORDERS AND FOR REPAIRING SPORTS INJURY, AND METHOD FOR MAKING SAME

(30) Priorité: 22.09.2005 FR 0509724
(43) Date de publication de la demande: 04.06.2008
(73) Titulaire: THUASNE, 92300 Levallois-Perret (FR)
(72) Inventeur: CHARDON-BRAS, Maryvonne, F-34070 Montpellier (FR); COURTET, François, F-42660 St Regis du Coin (FR)
(74) Mandataire: Jacobson, Claude
(86) Numéro de dépôt international: PCT/FR2006/002176
(87) Numéro de publication internationale: WO 2007/034090

(56) Documents cités:
- DE-U1- 29 618 426
- US-A- 4 377 159
- US-A- 5 356 372

## Description

La présente invention concerne un dispositif de traitement d'affections tissulaires cutanées et sous-cutanées et de réparation sportive, du type comprenant une première nappe de matériau souple, et une structure saillante solidaire de la nappe et comprenant une pluralité d'éléments saillants discrets, lesdits éléments saillants comprenant des morceaux de mousses espacés les uns des autres et solidaires de ladite première nappe.

Il existe différents dispositifs connus de ce type, comme des plaques de mousse sur une des faces desquelles font saillie des formes semi-sphériques (DE-296 18 426 U1). Dans d'autres dispositifs connus, une surface textile ou un matériau souple porte des demi-sphères de rayon de courbure très faible, de 1 mm à 5 mm, constituées d'une matière relativement dure.

Or de tels dispositifs présentent des résultats thérapeutiques décevants et une mauvaise tolérance de la part des patients en raison d'allergies ou d'une absence de confort.

Le but de l'invention est d'améliorer l'efficacité du traitement des affections tissulaires cutanées et sous-cutanées au moyen d'un dispositif bien toléré par les patients.

A cet effet, l'invention a pour objet un dispositif du type précité, caractérisé en ce que les morceaux de mousse sont comprimés entre ladite première nappe et une deuxième nappe de matériau souple, les deux nappes adhérant entre elles entre les éléments saillants.

D'autres caractéristiques de ce dispositif sont décrites dans les revendications 2 à 15.

L'invention a encore pour objets:
- une bande de traitement d'affections tissulaires cutanées et sous-cutanées et de réparation sportive comprenant un dispositif tel que défini ci-dessus, bordé par deux lisières dépourvues d'éléments saillants ;
- un vêtement de traitement d'affections tissulaires cutanées et sous-cutanées et de réparation sportive, comprenant sur au moins une partie de sa surface intérieure, un dispositif tel que défini ci-dessus ; et
- un procédé de fabrication d'un dispositif tel que défini ci-dessus, caractérisé en ce qu'on fait défiler ladite première nappe sur un plan sensiblement horizontal, on fait tomber régulièrement les morceaux de mousse qui se fixent sur ladite première nappe qui défile, et on applique sur les morceaux de mousse ladite deuxième nappe de matériau souple de façon qu'elle comprime les morceaux de mousse et adhère à ladite première nappe entre ces morceaux.

Dans un mode de mise en ouvre, on fait avancer des bandes de mousse dans des goulottes parallèles qui débouchent dans une zone d'alimentation, et on coupe les bandes de mousse à intervalles réguliers pour former les morceaux de mousse qui tombent sur ladite première nappe qui défile.

L'invention sera mieux comprise à la lecture de la description qui va suivre, donnée uniquement à titre d'exemple et faite en se référant aux dessins annexés, sur lesquels :
- la figure 1 est une vue en coupe d'un dispositif suivant l'invention ;
- la figure 2 est une vue de dessus du dispositif de la figure 1 ;
- les figures 3 et 4 sont des vues en coupe d'un montage du dispositif en bandage autour d'un bras ;
- la figure 5 est une vue de dessus d'un autre dispositif suivant l'invention, en bande, la nappe de dessus étant vue en transparence ;
- la figure 6 est une vue en coupe du montage du dispositif de la figure 5 ;
- la figure 7 est une vue de dessus de l'objet de la figure 6, le bandage de contention étant retiré, la nappe de dessus étant vue en transparence ;
- la figure 8 est une vue en perspective de l'impact du dispositif sur le bras quand on retire le dispositif ;
- la figure 9 est une vue en perspective des jambes d'un patient avec l'impact d'un montage du dispositif en bandage autour de l'une des jambes quand on a enlevé le dispositif ;
- les figures 10A à 10C sont des vues en perspective décrivant le procédé de fabrication d'une chaussette incluant le dispositif selon l'invention ;
- la figure 11 est une vue schématique en coupe illustrant un procédé de fabrication du dispositif des figures 1 et 2 ;
- la figure 12 est une vue schématique en coupe d'un second procédé de fabrication du dispositif des figures 1 et 2 ;
- la figure 13 est une vue schématique du dessus d'une variante de dispositif suivant l'invention ; et
- la figure 14 est une vue en coupe du dispositif dans un mode de réalisation particulier où les morceaux sont composés de deux mousses différentes.

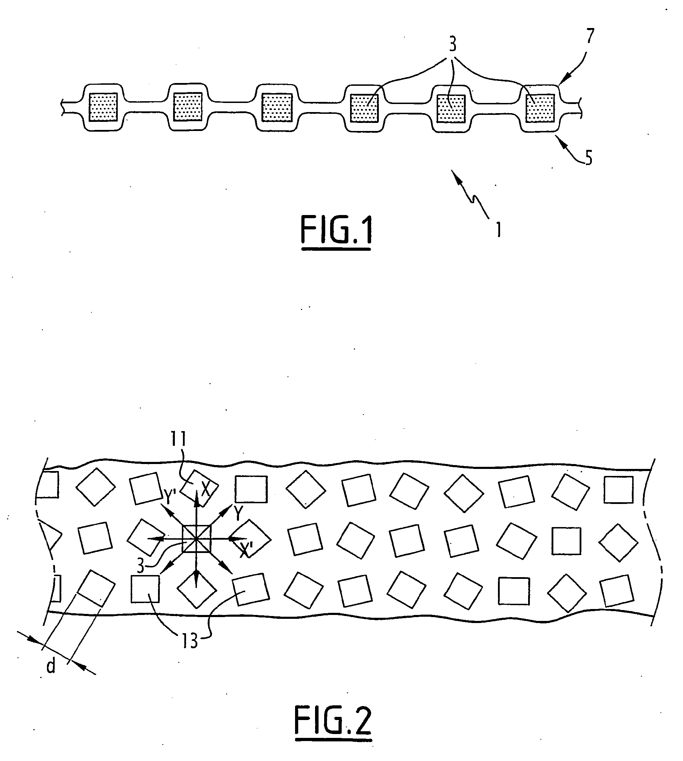

Le dispositif 1 représenté sur les figures 1 et 2 est destiné à être posé sur la peau sous un bandage de compression ou de contention, pour le traitement d'affections tissulaires cutanées ou sous-cutanées et en particulier pour le traitement de divers types d'oedèmes ou d'infiltrations tissulaires excessives. Dans ce cas, le dispositif est dit réducteur. Le dispositif peut également être utilisé pour la récupération sportive après l'effort. Le dispositif 1 comprend essentiellement des pavés de mousse 3 espacés les uns des autres, comprimés entre deux nappes de matériau souple 5 et 7. Les deux nappes sont adhésives entre elles entre les pavés de mousse.

Dans le mode de réalisation représenté, les pavés sont issus d'une plaque en mousse de latex coulé pouvant s'expanser librement de façon à développer une mousse légère à cellules ouvertes. La mousse est très élastique de façon à s'adapter à toutes les surfaces anatomiques sur lesquelles elle est pressée. L'indentation de la mousse peut varier suivant les pathologies et la zone corporelle où est appliqué le dispositif 1, comme le montre le tableau ci-dessous. En règle générale, l'indentation est comprise entre 200 et 250 N mais elle peut être plus élevée sur des peaux saines, pour un usage de réparation sportive. La densité de la mousse peut varier dans une fourchette de 13 à 100kg/m³.

Comme il est bien connu, l'indentation est une méthode de mesure de la dureté par indentation des matériaux alvéolaires souples. La valeur d'indentation est la force appliquée pour une pénétration de 40% d'un pénétreur, dans des conditions normalisées décrites dans la norme ISO2439 de septembre 1997.

Valeurs de l'indentation de la mousse en fonction du type de pathologie

| | |
|---|---|
| Indentation faible | Oedèmes de texture souple sur une peau fragile ou particulièrement douloureux : |
| | - Oedèmes post chirurgicaux (orthopédie...) |
| | - Oedèmes après lipoaspiration |
| | - Oedèmes traumatiques (entorse) |
| | - Oedèmes cardiaques ou rénaux qui retentissent sur les membres inférieurs |
| | - Oedèmes inflammatoires (algodystrophie gonarthrose...) |
| | - Oedèmes allergiques |
| | - Oedèmes des brûlures en phase aiguë |
| Indentation moyenne - | - Oedèmes de stases |
| | - Oedèmes d'insuffisance veineuse, aiguë ou chronique (phlébite, hypodermite,) |
| | - Oedèmes graisseux, cellulite, surcharge pondérale, oedèmes de cause neurologique périphérique (plexiteradique) |
| | - Oedèmes de cause centrale : hémiplégie, paraplégie, etc |
| | - Lymphoedèmes débutants |
| Indentation élevée | - Lymphoedèmes constitués |
| | - Eléphantiasis |
| | - Tous les tissus fibroses sains |
| | - Toutes les cicatrices chéloïdes (si elles ne sont pas inflammatoires) |
| | - Les ulcères, ni infectes ni inflammatoires |
| | - Les dermites ocres |

Valeurs de l'indentation de la mousse en fonction de la zone corporelle

| | |
|---|---|
| Indentation faible | Tous les reliefs osseux apparents (tendon d'achille, crête tibiale, clavicule etc....) et toutes les zones ou les ganglions lymphatiques, sont palpables |
| | - le genou |
| | - le cou de pied |
| | - le pli de l'aine |
| | - la face antéro-interne de la cuisse |
| | - la face antero-interne du bras |
| | - le coude |
| | - le creux axillaire etc... |
| Indentation élevée | - dessus du pied |
| | - arr des malléoles ext et int |
| | - face externe de la cuisse, de la hanche, fesses, dos en général le pubis et pratiquement toute la jambe (sauf de la crête tibiale) |
| | - le dos de la main |
| | - l'avant bras |
| | - la face ext et post du bras |
| | - les orteils et les doigts de pied |

La dimension et la forme des pavés varient suivant la pathologie et la zone corporelle sur laquelle le dispositif est pressé. Dans le mode de réalisation représenté, les morceaux de mousse ont une forme de pavés réguliers de hauteur h et dont la section a sensiblement la forme d'un carré, de côté d. Leur hauteur h varie de préférence entre 8 et 12 mm et la surface de la section varie de 25 mm², pour les doigts et les orteils, à 900 mm² pour le dos ou l'abdomen, en passant par 100 mm² pour la main et 400 mm² pour le bras ou la cuisse. Dans un autre mode de réalisation, les pavés sont des parallélépipèdes en forme de languettes allongées dont l'une des dimensions est très supérieure aux autres.

Le matériau souple utilisé pour les nappes 5 et 7 peut être par exemple un non tissé en polyester adhésivé d'une densité de 50g/m² ayant une élasticité intrinsèque dans une direction. En variante, il peut s'agir d'un non tissé en polyuréthane dans deux directions orthogonales l'une à l'autre ou d'un tissu élastique multidirectionnel.

La figure 2 montre la répartition des pavés de mousse les uns par rapport aux autres dans le plan de l'une des deux nappes. Les pavés de mousse sont sensiblement alignés selon des rangées régulières parallèles à une direction X, mais leur orientation est irrégulière par rapport à l'axe de symétrie de chaque cube perpendiculaire au plan de la nappe.

Chaque pavé 3 se trouve à une distance comprise entre d/2 et d d'un premier groupe de quatre pavés 11 immédiatement voisins et à une distance de l'ordre de 2d d'un second groupe de quatre pavés 13 immédiatement voisins. Les morceaux 3 et 11 sont sensiblement alignés suivant la direction X et suivant une direction X' sensiblement orthogonale à X, et les morceaux 3 et 13 sont alignés dans deux directions Y et Y' sensiblement orthogonales entre elles et inclinées à environ 45° par rapport aux directions X et X'.

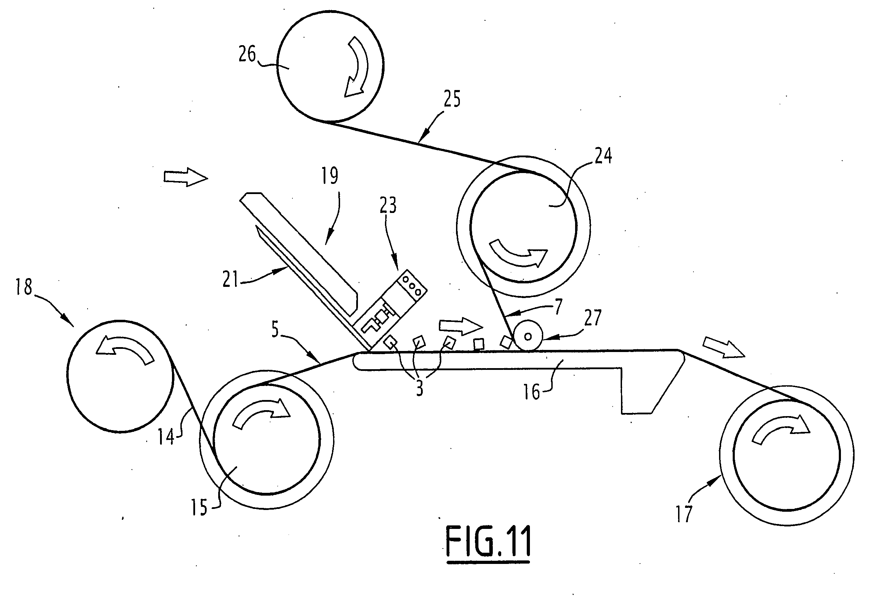

Cette disposition peut être obtenue par le procédé de fabrication décrit par la figure 11. La première nappe 5 de matériau souple adhésivée, revêtue d'une feuille de protection provisoire 14, et enroulée sur une bobine d'alimentation 15, est étendue sur un support plan horizontal 16 et défile horizontalement, tirée par une bobine de réception 17. En amont du support 16, la feuille 14 est enroulée sur une autre bobine 18. Une zone d'alimentation 19 en pavés est définie à l'extrémité amont du support 16. Cette zone d'alimentation contient plusieurs bandes de mousse alignées dans des goulottes parallèles et qui sont découpées en pavés par une guillotine 23.

Les pavés tombent à une fréquence réglable, de même que leur hauteur de chute est réglable. La fréquence de chute détermine l'espacement des cubes sur la nappe support 5 qui défile. La hauteur de chute détermine l'orientation des pavés, qui sera d'autant moins régulière que la hauteur sera grande.

Les pavés, en tombant; se fixent sur la nappe 5 adhésive. La deuxième nappe de matériau souple 7 déroulée d'une bobine 24, est également adhésivée et protégée provisoirement par une feuille 25, laquelle est récupérée sur une bobine 26. La nappe 7 est alors appliquée sur les pavés de mousse par un rouleau de calandrage 27. Comprimant les pavés de mousse 3, elle adhère à la première nappe support 5 entre ceux-ci.

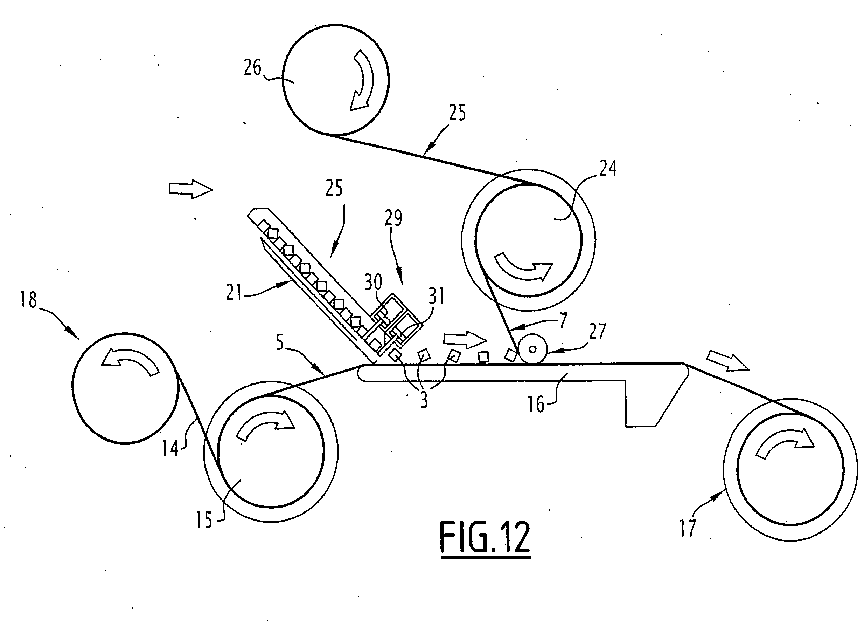

Dans un autre mode de réalisation (figure 12), la zone d'alimentation contient des pavés de mousse prédécoupés dans les goulottes 21 et sont distribués par un distributeur-doseur 29, par exemple à deux portes 30, 31 à mouvement alterné.

Les procédés décrits ci-dessus sont adaptés pour permettre la fabrication du dispositif 1 sous la forme d'une plaque par exemple d'une longueur de 5 mètres et d'une largeur de 30 cm, ou alors d'une bande de même longueur et d'une largeur de 10 cm.

Dans les deux cas, morceau de plaque découpé ou bande, le dispositif 1 peut être retenu contre la peau à l'aide d'une bande de contention, par exemple en FLEXIDEAL^{®}, qui maintient le dispositif contre la peau, ou bien par un bandage de compression, par exemple en BIFLEX^{®}, qui en plus de maintenir le dispositif, exerce une pression, plus ou moins forte, comprimant le dispositif contre la peau. Le type de bande utilisée rigide ou élastique pour le bandage réducteur dépend de la pression requise par la pathologie traitée.

La plaque est destinée à être découpée par un praticien qui va l'adapter à la surface du tissu à traiter.

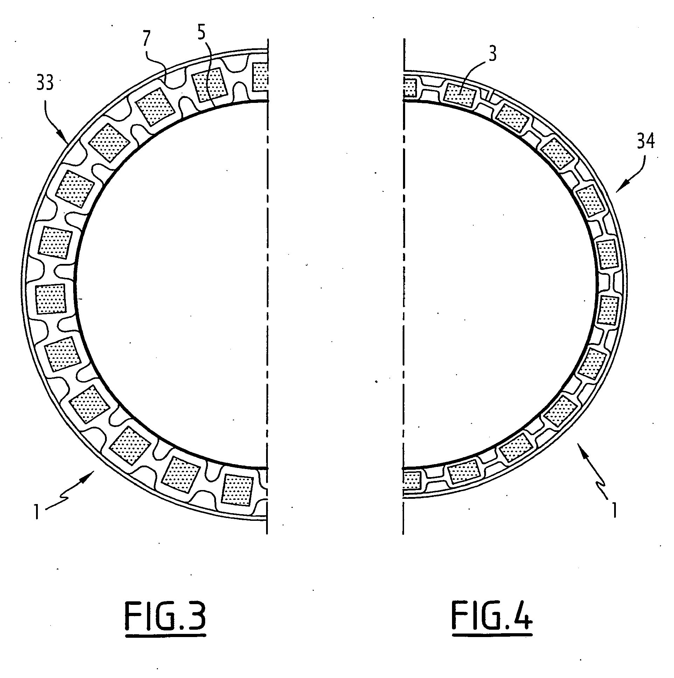

On voit sur la figure 3 une utilisation du dispositif en plaque sur un bras, sans compression notable. Le dispositif 1 est retenu par un pansement sous forme de filet circulaire 33. Ce montage agit très rapidement sur les oedèmes d'origine vasculaire qui sont la plupart du temps très souples, ainsi que sur les lymphoedèmes surtout liquidiens.

On voit sur la figure 4 une vue en coupe d'une utilisation du même dispositif sur un bras, avec compression. Dans ce cas, le dispositif 1 est maintenu par un bandage compressif 34, qui maintient une pression substantielle sur les cubes de mousse, beaucoup plus comprimés. Cette utilisation du dispositif est pratiquée dans les cas de lymphoedèmes très fibrosés.

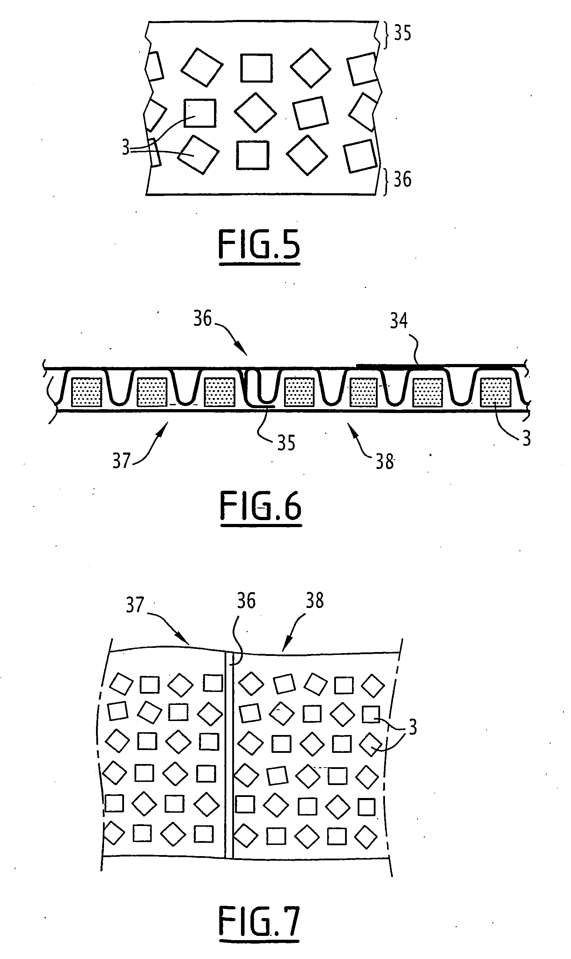

La bande (figure 5) est destinée à être utilisée pour réaliser un bandage autour d'un membre. Dans le cas de la bande, le procédé de fabrication aménagera deux lisières 35 et 36 le long de la bande, dépourvues de pavés de mousse. Ces lisières sont utilisées lors d'un bandage sur un membre, comme cela est représenté sur les figures 6 et 7. L'intérêt de la bande est de pouvoir s'adapter à la diminution de volume qu'elle génère, et de pouvoir, par sa simplicité, être utilisée par le patient lui-même. La lisière sert de guidage à la juxtaposition des différents tours de bandages. La figure 6 représente en coupe deux tours de bandage 37 et 38. La portion de lisière 35 du tour de bandage 37 sert de guidage pour le tour de bandage 38, dont la portion de lisière 36 vient s'appuyer sur les pavés adjacents du tour 37. La figure 7 montre en vue schématique de dessus que le bandage ainsi réalisé sur le bras est sensiblement homogène en ce qui concerne la répartition des pavés de mousse.

Le dispositif 1 peut aussi être utilisé sous la forme d'un vêtement contentif. L'avantage du vêtement contentif est de rendre le traitement beaucoup plus supportable pour le patient. Le patient sera ainsi plus enclin à suivre son traitement, qui gagnera donc en efficacité.

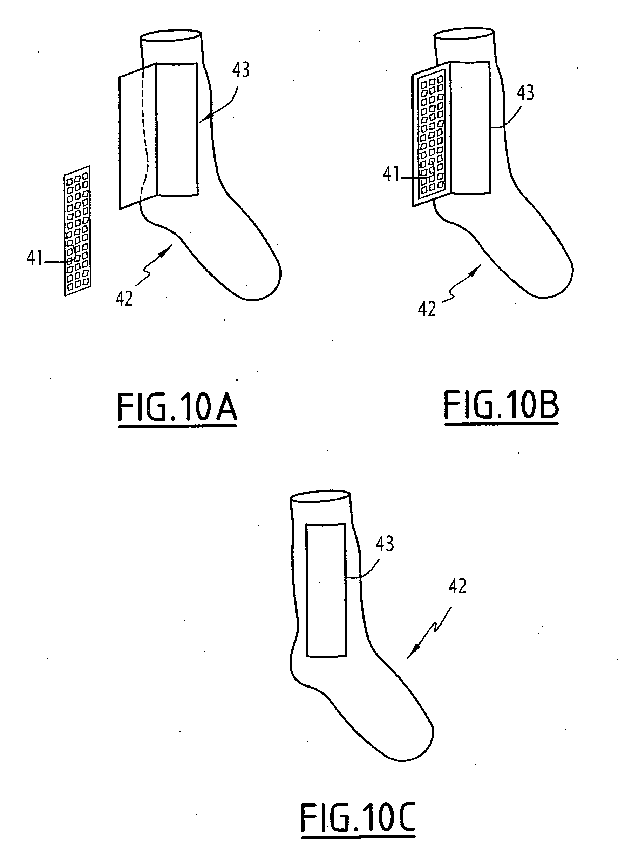

Un mode de réalisation d'un vêtement associé au dispositif 1 est illustré sur les figures 10A à 10C : le dispositif 1, sous la forme d'un morceau 41 de plaque ou de bande découpé, est inséré dans un vêtement 42 qui a été découpé selon une génératrice 43. Le morceau 41 est cousu à l'intérieur du vêtement, à l'endroit où l'on veut appliquer le traitement. Le vêtement est enfin recousu selon la génératrice 43.

Le vêtement peut être en particulier une chaussette, un bas ou un collant pour sportif, ou un panty destiné à diverses utilisations. Le panty peut également servir à un traitement contre la cellulite.

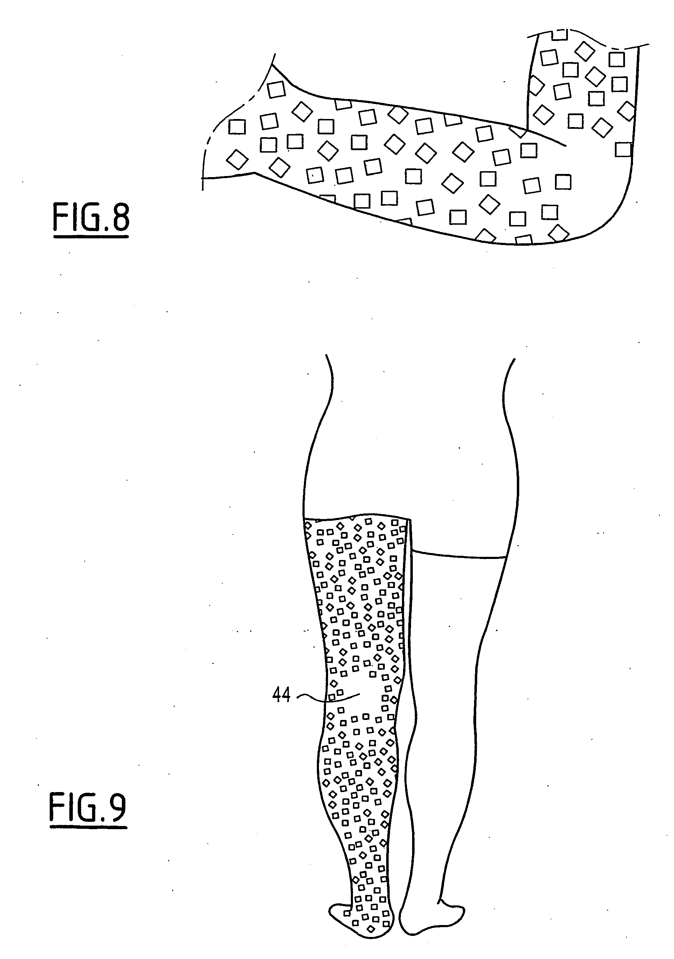

On évitera, par précaution, de couvrir l'intérieur du vêtement avec le dispositif dans les régions des articulations comme le genou et le creux poplité 44, dans le cas d'un bas montant au-dessus du genou (figure 9).

Ce type de vêtement peut être réalisé sur mesure, en étant avantageusement dessiné par CAO (Conception Assistée par Ordinateur) puis coupé et cousu, ou fabriqué à l'aide d'une machine à tricoter circulaire à commande numérique pilotée par un logiciel pouvant doser la compression par son adaptabilité aux mesures du patient stockées en mémoire.

Le bandage réducteur est d'autant plus efficace que la pression du dispositif contre la peau est grande, qu'il est porté longtemps et qu'une activité musculaire y est associée.

La pression peut être augmentée par un montage compressif, et/ou par une activité musculaire du membre concerné par le bandage.

Le temps durant lequel le patient porte le bandage peut être allongé car le dispositif est bien toléré et simple à utiliser. La tolérance du patient au dispositif, liée à un bandage peu compressif ou à un vêtement sur mesure, permet au patient par exemple de porter le montage durant son sommeil.

Les figures 9 et 10 montrent sur un bras (figure 9) et sur une jambe (figure 10), après avoir retiré le bandage réducteur, les marques des pavés sur la peau. Les arêtes des pavés de mousse comprimés contre la peau agissent par un effet de cisaillement sur la peau, la massent, permettant d'accélérer la micro-circulation de surface au niveau du réseau cutané superficiel, et dans le cas d'oedèmes lymphatiques et d'amorcer l'assouplissement des éléments sous-jacents de la peau. Cet effet de cisaillement consiste à mobiliser des tissus les uns par rapport aux autres, à favoriser des plans de glissement afin d'atteindre les zones plus profondes du réseau cutané. Grâce aux différences de distances entre morceaux de mousse immédiatement voisins et à l'élasticité des nappes 5 et 7, des pressions différentes exercées sur le tissu sous-jacent ont pour effet de casser la raideur et l'immobilité de celui-ci.

L'effet de cisaillement rend ainsi beaucoup plus efficace la pression qui entraîne le drainage des liquides vers les collecteurs superficiels puis profonds du réseau lymphatique. Cet effet de pression est rendu efficace par la faible distance qui sépare chaque morceau de mousse du premier groupe de morceaux voisins, tandis que l'effet de cisaillement, lié à la liberté de déplacement des pavés, est favorisé par la grande distance qui sépare chaque pavé du second groupe de pavés voisins, et par l'élasticité des nappes 5 et 7.

Ainsi, le dispositif selon l'invention agit selon un double effet, de cisaillement et d'auto-massage drainant, particulièrement bénéfique pour la circulation dans la région cutanée et sous-cutanée.

Cette double action essentielle est d'autant plus efficace que le traitement est bien toléré par les patients grâce aux procédés de fabrication entre deux nappes. Le dispositif peut aussi être utilisé la nuit, sous la forme de vêtement ou de bandage peu compressif, ce qui favorise la bonne observance du traitement par le patient.

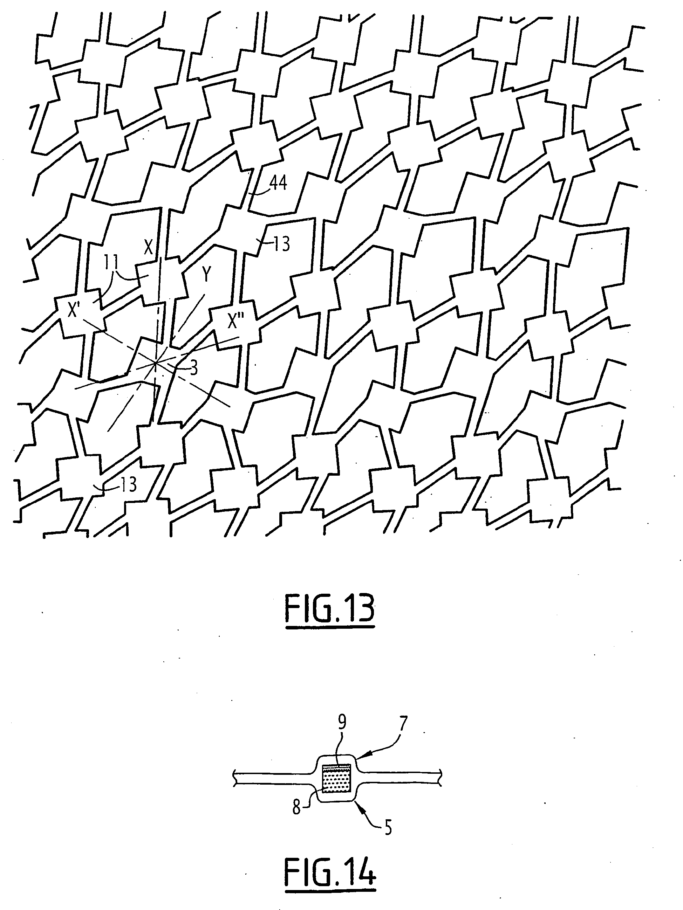

Dans un autre mode de réalisation, représenté sur la figure 14, chaque morceau de mousse est composé de deux éléments superposés constitués de mousses différentes, le premier élément 8 en une mousse à indentation élevée du côté d'une des nappes de matériau souple 5, et le deuxième élément 9 en une mousse à indentation faible, collée sur l'élément 8 du côté de l'autre nappe de matériau souple 7. Les éléments 8 et 9 peuvent être collés l'un sur l'autre. En variante, par un procédé de moussage approprié, on peut obtenir des pavés ayant une variation continue d'indentation d'une face à l'autre.

L'avantage de ce mode de réalisation consiste à améliorer la répartition de l'effort de contention/compression, celui-ci étant appliqué sur la face à indentation faible, et ce sans rendre les pavés plus agressifs pour la peau du patient.

La figure 13 montre un autre mode de réalisation où les pavés sont disposés en quinconce d'une rangée suivant la direction X à la suivante, chaque pavé étant relié à quatre de ses plus proches voisins par un lien de mousse 44. Cette disposition des pavés peut être obtenue par une découpe à l'emporte-pièce dans une plaque de mousse.

Dans ce cas, chaque pavé 3 comporte six voisins proches 11, alignés suivant trois directions X, X' et X", et deux voisins nettement plus éloignés 13, ceux-ci étant alignés suivant une direction Y intermédiaire entre les directions X et X".

## Revendications

1. Dispositif de traitement d'affections tissulaires cutanées et sous-cutanées et de réparation sportive, du type comprenant une première nappe de matériau souple (5), et une structure saillante solidaire de la nappe et comprenant une pluralité d'éléments saillants discrets, lesdits éléments saillants comprenant des morceaux de mousse (3) espacés les uns des autres et solidaires de ladite première nappe (5), **caractérisé en ce que** les morceaux de mousse sont comprimés entre ladite première nappe (5) et une deuxième nappe (7) de matériau souple, les deux nappes adhérant entre elles entre les éléments saillants.

2. Dispositif selon la revendication 1, **caractérisé en ce que** la mousse composant les morceaux de mousse (3) est une mousse à cellules ouvertes.

3. Dispositif selon la revendication 1 ou 2, **caractérisé en ce que** sur au moins une fraction de la surface du dispositif, la distance qui sépare chaque morceau de mousse (3) d'un second groupe (13) de morceaux de mousse immédiatement voisins est plusieurs fois supérieure à la distance qui sépare ce morceau de mousse d'un premier groupe (11) de morceaux de mousse immédiatement voisins.

4. Dispositif selon la revendication 3, **caractérisé en ce que** sur ladite fraction de la surface, chaque morceau de mousse (3) se trouve à une distance de l'ordre d'une fraction de d ou voisine de d dudit premier groupe (11) de morceaux de mousse immédiatement voisins et à une distance de l'ordre d'un multiple de d dudit second groupe (13) de morceaux de mousse immédiatement voisins, d étant la dimension moyenne d'un morceau de mousse dans le plan de ladite première nappe.

5. Dispositif selon la revendication 3 ou 4, **caractérisé en ce que** les morceaux dudit premier groupe (11) se trouvent dans des premières directions, et **en ce que** les morceaux dudit second groupe (13) se trouvent dans au moins une seconde direction intermédiaire entre lesdites premières directions.

6. Dispositif selon la revendication 5, **caractérisé en ce que** lesdites premières directions (X, X') sont sensiblement orthogonales entre elles, et **en ce que** les morceaux dudit second groupe (13) se trouvent dans deux secondes directions (Y, Y') sensiblement orthogonales entre elles.

7. Dispositif selon l'une quelconque des revendications précédentes, **caractérisé en ce que** les morceaux de mousse (3) sont des parallélépipèdes.

8. Dispositif selon la revendication 7, **caractérisé en ce que** les parallélépipèdes ont, en plan, deux dimensions voisines l'une de l'autre.

9. Dispositif selon la revendication 7, **caractérisé en ce que** les parallélépipèdes ont une forme de languettes dont l'une des dimensions est très supérieure aux autres.

10. Dispositif selon l'une quelconque des revendications précédentes, **caractérisé en ce que** la ou chaque nappe (5, 7) est faite d'un non tissé ayant une élasticité intrinsèque dans au moins une direction.

11. Dispositif selon l'une quelconque des revendications 1 à 9, **caractérisé en ce que** la ou chaque nappe (5, 7) est faite d'un tissu élastique, notamment multidirectionnel.

12. Dispositif selon l'une quelconque des revendications précédentes, **caractérisé en ce que** la mousse composant les morceaux de mousse a une indentation d'une valeur comprise entre 200 N et 250 N environ.

13. Dispositif selon l'une quelconque des revendications précédentes, **caractérisé en ce que** les éléments saillants sont reliés entre eux par des liens (44) venus de matière avec eux.

14. Dispositif selon l'une quelconque des revendications précédentes, **caractérisé en ce que** chaque morceau de mousse comporte une première couche extrême à indentation forte et une seconde couche extrême à indentation faible.

15. Dispositif selon la revendication 14, **caractérisé en ce que** chaque morceau de mousse est composé de deux éléments superposés constitués de mousses différentes et solidaires entre eux, à savoir un premier élément (8) en mousse à indentation faible et un deuxième élément (9) en mousse à indentation élevée.

16. Bande de traitement d'affections tissulaires cutanées et sous-cutanées et de récupération sportive, **caractérisée en ce qu'**elle comprend un dispositif selon l'une quelconque des revendications 1 à 15, bordé par deux lisières (31, 33) dépourvues d'éléments saillants.

17. Vêtement (42) de traitement d'affections tissulaires cutanées et sous-cutanées et de récupération sportive, **caractérisé en ce qu'**il comprend, sur au moins une partie de sa surface intérieure, un dispositif selon l'une quelconque des revendications 1 à 15.

18. Procédé de fabrication d'un dispositif selon l'une quelconque des revendications 1 à 12, **caractérisé en ce qu'**on fait défiler ladite première nappe (5) sur un plan sensiblement horizontal (16), on fait tomber régulièrement les morceaux de mousse (3) qui se fixent sur ladite première nappe qui défile, et on applique sur les morceaux de mousse ladite deuxième nappe (7) de matériau souple de façon qu'elle comprime les morceaux de mousse et adhère à ladite première nappe entre ces morceaux.

19. Procédé de fabrication selon la revendication 18, **caractérisé en ce qu'**on fait avancer des bandes de mousse dans des goulottes parallèles qui débouchent dans une zone d'alimentation, et on coupe les bandes de mousse à intervalles réguliers pour former les morceaux de mousse qui tombent sur ladite première nappe (5) qui défile.

## Claims

1. Device for treating disorders of the cutaneous and sub-cutaneous tissue and repairing sports injury, of the type comprising a first web of flexible material (5) and a projecting structure joined to the web and comprising a plurality of discrete projecting elements, said projecting elements comprising pieces of foam (3) spaced apart from one another and joined to said first web (5), **characterised in that** the pieces of foam are compressed between said first web (5) and a second web (7) of flexible material, the two webs adhering to one another between the projecting elements.

2. Device according to claim 1, **characterised in that** the foam used to make the pieces of foam (3) is a foam with open cells.

3. Device according to claim 1 or 2, **characterised in that** the distance separating each piece of foam (3) from a second group (13) of immediately adjacent pieces of foam is several times greater than the distance separating this piece of foam from a first group (11) of immediately adjacent pieces of foam on at least a fraction of the surface of the device.

4. Device according to claim 3, **characterised in that**, on said fraction of the surface, each piece of foam (3) is disposed at a distance in the order of a fraction of d or close to d from said first group (11) of immediately adjacent pieces of foam and at a distance in the order of a multiple of d from said second group (13) of immediately adjacent pieces of foam, d being the mean dimension of a piece of foam in the plane of said first web.

5. Device according to claim 3 or 4, **characterised in that** the pieces of said first group (11) are disposed in first directions, and the pieces of said second group (13) are disposed in at least one second intermediate direction between said first directions.

6. Device according to claim 5, **characterised in that** said first directions (X, X') are substantially orthogonal to one another, and the pieces of said second group (13) are disposed in two second directions (Y, Y') substantially orthogonal to one another.

7. Device according to any one of the preceding claims, **characterised in that** the pieces of foam (3) are parallelepipeds.

8. Device according to claim 7, **characterised in that** the parallelepipeds have two dimensions similar to one another in plan view.

9. Device according to claim 7, **characterised in that** the parallelepipeds are based on the shape of tongues, one of the dimensions of which is very much bigger than the others.

10. Device according to any one of the preceding claims, **characterised in that** the or each web (5, 7) is made from a non-woven fabric with an intrinsic elasticity in at least one direction.

11. Device according to any one of claims 1 to 9, **characterised in that** the or each web (5, 7) is made from a fabric that is elastic, in particular in multiple directions.

12. Device according to any one of the preceding claims, **characterised in that** the foam used to make the pieces of foam has an indentation with a value ranging between approximately 200 N and 250 N.

13. Device according to any one of the preceding claims, **characterised in that** the projecting elements are joined to one another by integral links (44) in the material between them.

14. Device according to any one of the preceding claims, **characterised in that** each piece of foam comprises a first end layer with a pronounced indentation and a second end layer with a slight indentation.

15. Device according to claim 14, **characterised in that** each piece of foam is made up of two superposed elements made from different foams and joined to one another, namely a first element (8) made from foam with a slight indentation and a second element (9) made from foam with a pronounced indentation.

16. Strap for treating disorders of the cutaneous and sub-cutaneous tissue and repairing sports injury, **characterised in that** it comprises a device according to any one of claims 1 to 15, edged with two selvedges (31, 33) with no projecting elements.

17. Garment (42) for treating disorders of the cutaneous and sub-cutaneous tissue and repairing sports injury, **characterised in that** it comprises a device according to any one of claims 1 to 15 on at least a part of its internal surface.

18. Method of manufacturing a device according to any one of claims 1 to 12, **characterised in that** said first web (5) is fed along a substantially horizontal plane (16), the pieces of foam (3) are dropped regularly and adhere to said first web as it advances, and the second web (7) of flexible material is applied to the pieces of foam so that it compresses the pieces of foam and adheres to said first web between these pieces.

19. Manufacturing method according to claim 18, **characterised in that** the strips of foam are fed in parallel chutes which open into a supply zone, and the strips of foam are cut at regular intervals to form the pieces of foam which drop onto said first web (5) as it advances.

## Patentansprüche

1. Vorrichtung zur Behandlung von kutanen und subkutanen Gewebekrankheiten und zur Reparatur von Sportverletzungen, des Typs, der ein erstes Tuch aus weichem Material (5) und eine einstückig mit dem Tuch verbundene und eine Mehrzahl von diskreten hervorspringenden Elementen aufweisende Struktur umfasst, wobei die hervorspringenden Elemente Schaumstoffstücke (3) aufweisen, die voneinander beabstandet sind und mit dem ersten Tuch einstückig verbunden sind, **dadurch gekennzeichnet, dass** die Schaumstoffstücke zwischen dem ersten Tuch (5) und einem zweiten Tuch (7) aus weichem Material komprimiert sind, wobei die zwei Tücher zwischen den hervorspringenden Elementen aneinander haften.

2. Vorrichtung nach Anspruch 1, **dadurch gekennzeichnet, dass** der die Schaumstoffstücke (3) bildende Schaumstoff ein Schaumstoff mit offenen Poren ist.

3. Vorrichtung nach Anspruch 1 oder Anspruch 2, **dadurch gekennzeichnet, dass** auf mindestens einem Bruchteil der Fläche der Vorrichtung der Abstand, der jedes Schaumstoffstück von einer zweiten Gruppe von unmittelbar benachbarten Schaumstoffstücken trennt, mehrfach größer ist als der Abstand, der dieses Schaumstoffstück von einer ersten Gruppe (11) von unmittelbar benachbarten Schaumstoffstücken trennt.

4. Vorrichtung nach Anspruch 3, **dadurch gekennzeichnet, dass** auf dem Bruchteil der Fläche jedes Schaumstoffstück sich einem Abstand in der Größenordnung eines Bruchteils von d oder in der Nähe von d von der ersten Gruppe (11) unmittelbar benachbarter Schaumstoffstücke und in einem Abstand in der Größenordnung eines Vielfachen von d von der zweiten Gruppe (13) unmittelbar benachbarter Schaumstoffstücke befindet, wobei d die mittlere Abmessung eines Schaumstoffstückes in der Ebene des ersten Tuches ist.

5. Vorrichtung nach Anspruch 3 oder 4, **dadurch gekennzeichnet, dass** die Stücke der ersten Gruppe (11) sich in ersten Richtungen befinden und dass die Stücke der zweiten Gruppe (13) sich in mindestens einer zweiten Richtung zwischen den ersten Richtungen befinden.

6. Vorrichtung nach Anspruch 5, **dadurch gekennzeichnet, dass** die ersten Richtungen (x, x') im Wesentlichen orthogonal zueinander sind und dass die Stücke der zweiten Gruppe (13) sich in zwei zweiten Richtungen (y, y'), die im Wesentlichen orthogonal zueinander sind, befinden.

7. Vorrichtung nach einem beliebigen der vorhergehenden Ansprüche, **dadurch gekennzeichnet, dass** die Schaumstoffstücke (3) als Parallelepiped ausgebildet sind.

8. Vorrichtung nach Anspruch 7, **dadurch gekennzeichnet, dass** die Parallelepipede in eier Ebene zwei zueinander benachbarte Abmessungen aufweisen.

9. Vorrichtung nach Anspruch 7, **dadurch gekennzeichnet, dass** die Parallelepipede die Form einer Zunge haben, bei der eine der Abmessungen sehr viel größer als die anderen ist.

10. Vorrichtung nach einem beliebigen der vorhergehenden Ansprüche, **dadurch gekennzeichnet, dass** das oder jedes Tuch (5, 7) aus einem Vlies hergestellt ist, das eine Eigenelastizität in mindestens eine Richtung aufweist.

11. Vorrichtung nach einem beliebigen der Ansprüche 1 bis 9, **dadurch gekennzeichnet, dass** das oder jedes Tuch (5, 7) aus einem elastischen Stoff, insbesondere einem in mehrere Richtungen elastischen Stoff, hergestellt ist.

12. Vorrichtung nach einem beliebigen der vorhergehenden Ansprüche, **dadurch gekennzeichnet, dass** der Schaumstoff, der die Schaumstoffstücke bildet, eine Eindruckhärte eines Wertes zwischen ungefähr 200 N und 250 N hat.

13. Vorrichtung nach einem beliebigen der vorhergehenden Ansprüche, **dadurch gekennzeichnet, dass** die hervorspringenden Elemente unter sich über Bindungen (44) verbunden sind, die aus einem Material mit den Elementen hergestellt sind.

14. Vorrichtung nach einem beliebigen der vorhergehenden Ansprüche, **dadurch gekennzeichnet, dass** jedes Schaumstoffstück eine erste äußerste Schicht mit großer Endruckhärte und eine zweite äußerste Schicht mit geringer Eindruckhärte aufweist.

15. Vorrichtung nach Anspruch 14, **dadurch gekennzeichnet, dass** jedes Schaumstoffstück aus zwei übereinanderliegenden Teilen zusammengesetzt ist, die aus unterschiedlichen miteinander verbundenen Schaumstoffen gebildet sind, nämlich einem ersten Element (8) aus Schaumstoff mit geringer Eindruckhärte und einem zweiten Element aus Schaumstoff mit erhöhter Eindruckhärte.

16. Bandage zur Behandlung von kutanen und subkutanen Gewebekrankheiten und zur Reparatur von Sportverletzungen, **dadurch gekennzeichnet, dass** sie eine Vorrichtung nach einem beliebigen der Ansprüche 1 bis 15 umfasst, die von zwei Randstreifen (31, 33) ohne hervorspringende Elemente eingefasst ist.

17. Bekleidungsstück (42) zur Behandlung von kutanen und subkutanen Gewebekrankheiten und zur Reparatur von Sportverletzungen, **dadurch gekennzeichnet, dass** sie auf mindestens einem Teil der Innenfläche eine Vorrichtung nach einem beliebigen der Ansprüche 1 bis 15 umfasst.

18. Verfahren zur Herstellung einer Vorrichtung nach einem beliebigen der Ansprüche 1 bis 12, **dadurch gekennzeichnet, dass** man das erste Tuch (5) auf einer im Wesentlichen horizontalen Ebene (16) vorbeifahren lässt, man regelmäßig Schaumstoffstücke (3) darauffallen lässt, die sich auf dem ersten Tuch, das vorbeifährt, ansiedeln, und man auf die Schaumstoffstücke das zweite Tuch (7) aus weichem Material auf eine solche Weise aufbringt, dass es die Schaumstoffstücke komprimiert und an dem ersten Tuch zwischen diesen Stücken anhaftet.

19. Verfahren zur Herstellung nach Anspruch 18, **dadurch gekennzeichnet, dass** man Bänder aus Schaumstoff in parallelen Rutschen fördert, die in eine Versorgungszone münden, und man die Bänder aus Schaumstoff in regelmäßigen Intervallen schneidet, um die Schaumstoffstücke zu bilden, die auf das erste Tuch (5), das vorbeifährt, fallen.
